# EUROPEAN PATENT SPECIFICATION

(11) **EP 0 276 661 B1**
(45) Date of publication and mention of the grant of the patent: **07.07.1993**
(21) Application number: 88100185.3
(22) Date of filing: 08.01.1988
(51) Int. Cl.: A61B 5/0402

(54) **Electrocardiographic electrode**
EKG-Elektrode
Electrode pour électrocardiographie

(30) Priority: 16.01.1987 JP 4575/87; 16.01.1987 JP 4577/87
(43) Date of publication of application: 03.08.1988
(73) Proprietor: FUKUDA DENSHI CO., LTD., Tokyo 113 (JP); KABUSHIKI KAISHA RIKEN, Chiyoda-ku Tokyo 102 (JP)
(72) Inventor: Ishi, Hiroyoshi, Kumagaya-shi Saitama-ken (JP); Suzuki, Kenzo, Kumagaya-shi Saitama-ken (JP); Kaneko, Misao, Kumagaya-shi Saitama-ken (JP); Kawamoto, Hiroshi, Kumagaya-shi Saitama-ken (JP); Shimizu, Chuji, Funabashi-shi Chiba-ken (JP); Onodera, Yasuaki, Hidaka-cho Irimagun Saitama-ken (JP)
(74) Representative: TER MEER STEINMEISTER & PARTNER GbR

(56) References cited:
- US-A- 3 681 136
- US-A- 3 841 312
- US-A- 3 882 853
- US-A- 3 989 036
- US-A- 4 490 005

## Description

The present invention relates to an electrocardiographic electrode comprising an electrode member having a head portion with a lead attached thereto, a neck portion having an engagement portion narrower than the head portion and capable of being engaged in an expansible member and a bottom portion formed under and broader than and supporting said neck portion, the outer surface of said synthetic resin electrode member being imparted with electric conductivity by a metal powder coating, the expansible member engagedly locked in said engagement portion of the neck portion, an adhesive film consisting of an adhesive, having a central opening and secured to said expansible member to be held in close contact with the skin of the living body.

Electricity is generated in the living body by the activity of the heart, brain, muscles, etc.

Particularly, electrocardiographic electrodes are used for diagnosing the heart by reducing a weak current that is induced on the skin of the living body using an external electrocardiograph.

To couple an input section of the electrocardiograph and the living body electrically, it is necessary to have electrocardiographic electrodes in close contact with the skin of the living body.

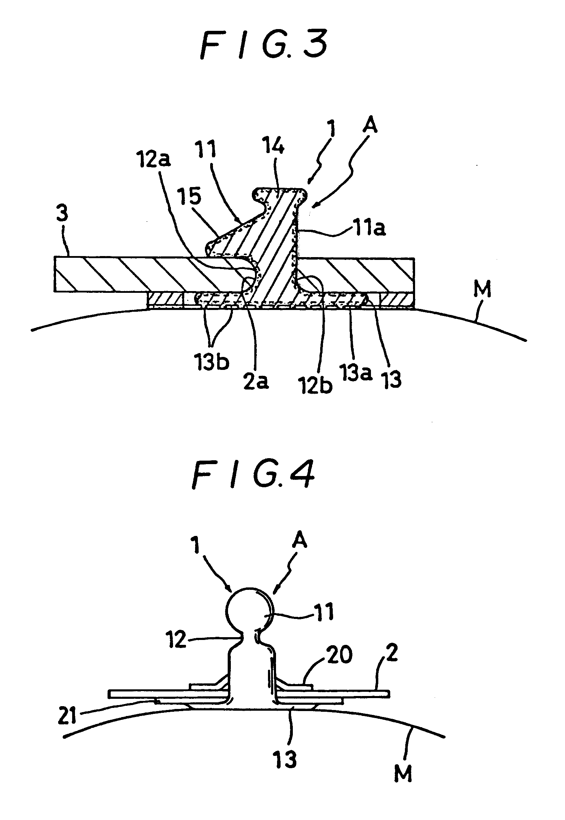

A prior art electrocardiographic electrode which is held in close contact with the skin of the living body, will now be explained with reference to Fig. 4. In the Figure, reference numeral 1 designates an electrode element made of a metal. An expansible member 2 is engaged in the electrode element 1. The expansible member 2 is secured to the electrode element 1 in a state clamped between upper and lower clamp members 20 and 21.

The electrode element 1 has a broad bottom portion 13 which is covered by a conductive cover member 7. The electrode element 1 derives an electrocardiographic electrode from the skin via the conductive cover member 7.

Such electrode member 1 for an electrocardiographic electrode, however, is made of a metal and is thus heavy. Therefore, it is not suited for being appliedly held on the skin. In addition, electrode characteristics are unsatisfactory because of high polarization voltage.

Further, the electrode element 1 has heretofore been manufactured by silver plating. Such method of manufacturing the electrode element by silver plating, however, is complicated in procedure as well as being dangerous and requiring high manufacturing cost.

Furthermore, the expansible member 2 engaged in the electrode element 1 requires two clamping members 20 and 21 for holding it in an engaged state.

The US-A-4 490 005 discloses an electrode as set out in the preamble of independent claims 1 and 2, which comprises an electrical connector for connecting an electrode to a lead. The particular problem underlying this invention is that a relative rotation between the lead an the electrode is wanted. Thus the inventor proposed a flat pad to be fixed to the surface to be detected, and a connector having a plug which may be inserted into an opening of the pad. The plug is rotatable within this opening.

US-A-3 989 036 discloses an electrocardiographic electrode. The electrode which gets in touch with the skin consists of an insulating member being coated by baking silver powder.

The electrode member is provided with a number of integral projections increasing the contact area between the electrode and the skin.

It is the object of the invention providing satisfactory electric conductivity between the skin of the patient and the lead extending from the electrode.

To comply with this object, the invention is characterized in that the coating is made from metal powder, the head portion has a straight, recessed portion, and that the neck portion has a straight portion continuous to and forming a straight surface with the straight recessed portion of the head portion, the bottom portion being in contact with the skin of the living body through gel-like electrolyte material and an electrolyte material cover, wherein the term "straight" qualifies the shape of the outline of a longitudinal section of the electrode member.

Alternatively, the invention is characterized in that the coating is made from metal powder, the head portion has a straight, recessed portion, and that the neck portion has a straight portion continuous to and forming a straight surface with the straight recessed portion of the head portion, the bottom portion being held in contact with the skin of a living body, the lower surface area thereof being irregular to increase the contact area with the skin, wherein the term "straight" qualifies the shape of the outline of a longitudinal section of the electrode member.

Since in the above construction according to the invention the neck portion of the electrode element is formed under and narrower than the head portion, the expansible member is reliably locked by engagement.

In addition, since the electrode element has a broad bottom portion, the area of its contact with the skin is increased to provide more satisfactory electric conductivity.

Further, since the head portion has the straight recessed portion and also the neck portion has the straight portion continuous to the straight recessed portion, satisfactory coating of even a small quantity of metal powder can be obtained.

Further, since the electrode element is made of a synthetic resin, it is reduced in weight. Still further, since its outer surface is coated with metal powder, it has electric conductivity.

Furthermore, since the adhesive film has a central opening and is secured to the expansible member engaged in the straight electrode member, the outer periphery of the electrocardiographic electrode is held in close contact with the skin of the living body.

### Brief Description of the Drawings

Fig. 1 is a perspective view showing one embodiment of the electrocardiographic electrode according to the invention;
Fig. 2 is a sectional view taken along line II-II in Fig. 2;
Fig. 3 is a sectional view showing a different embodiment of the electrocardiographic electrode; and
Fig. 4 is a view for explaining the prior art.

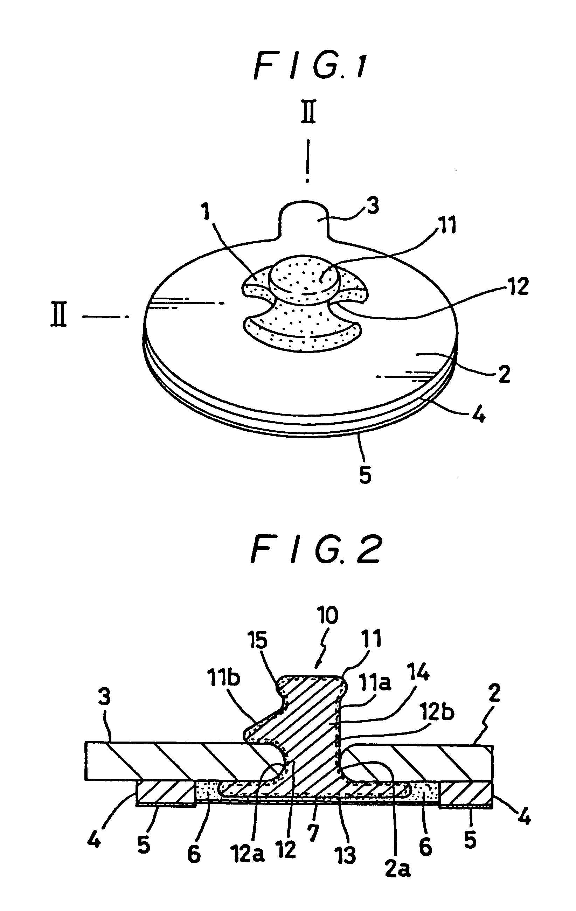

Fig. 1 is a perspective view showing an electrocardiographic electrode according to the invention. In the Figure, reference numeral 1 designates an electrode element. A circular expansible member 2 is engaged in the electrode element 1. The expansible member 2 has a manipulating portion 3 projecting from the edge to facilitate the attachment and detachment of the electrocardiographic electrode with respect to the skin.

An attachment member 4 is attached to the underside of the expansible member 2, and an adhesive film 5 is provided on the underside of the attachment member 2. The viscosity of the adhesive film 5 permits the electrocardiographic electrode to be held in close contact with the skin of a living body.

Fig. 2 is a sectional view showing one embodiment of the invention. The electrode element 1 has a head portion 11, to which a lead (not shown) is attached, a neck portion 12 formed under the head portion 11 and a bottom portion 13 formed under the neck portion 12. The synthetic resin material 14 is imparted with electric conductivity by coating the outer surface with powder of a metal, e.g., silver or an amorphous alloy, as coating agent 15.

The synthetic resin material 14 is made of a synthetic resin in order that it is suited for mass production for cost reduction, that it is reduced in weight to ensure that it is stably held fitted on the skin of a living body and that conduction is prevented to prevent the sense of coldness of the skin when it is attached thereto. Further, the outer surface of the synthetic resin material 14 is coated with powder 15 of metal in order that the effective area of the outer surface of the material 14 is increased with formation of irregularities, thus improving electrode characteristics.

A lead (not shown) is attached to the head portion 11. The head portion 11 has a straight recessed portion 11a, as shown in Fig. 2.

The neck portion 12 supporting the head portion 11 has an engaged portion 12a narrower than the head portion 11 and a straight portion 12b continuous to and forming a straight surface with the straight recessed portion 11a of the head portion. The engagement portion 12a is provided in order that the expansible member 2 is readily and reliably engaged without use of any clamping members.

The expansible member 2 is formed with a central small hole 2a. When the head portion 11 of the electrode element 1 is inserted through the small hole 2a from below, the small hole 2a is expanded as it is passed by an inclined portion 11b due to the expansibility of the expansible member 2. When the expansible member 2 is fitted up to the narrower neck portion 12, the small hole 2a is contracted by the restoring force due to its own expansibility.

Therefore, the expansible member 2 is readily engaged in the engagement portion 12a of the neck portion 11 of the electrode element and held clamped betwween the head portion 11 and bottom portion 13.

The head portion is formed with the straight recessed portion 11a and the neck portion is formed with the straight portion 12b continuous to and forming a straight surface with the recessed portion 11a in order that even a small quantity of metal powder can be satisfactorily coated and that satisfactory electric conductivity can be obtained.

Under the neck portion 12, the bottom portion 13 supporting the neck portion 12 is formed such that it is broader than the neck portion 12. Thus, the expansible member 2 engaged in the engagement portion 12a of the neck portion 12 is clamped between the head portion 11 and bottom portion 13.

The bottom surface 13a of the bottom portion 13, which is held in direct contact with the skin M of the living body, as shown in Fig. 3, has an irregular surface area 13b, thus increasing the contact area and stabilizing the electrode characteristics.

As shown in Fig. 2, near the bottom portion 13 of the electrode element 1 there are a gell-like electrolyte material 6 and an electrolyte material cover 7 covering the electrolyte material 6 and for being in electric contact with the skin. The electrolyte material 6 fills the space between the electrode element 1 and cover 7 to improve the electric conductivity.

As has been shown, in the above embodiment of the electrocardiographic electrode the outer surface of the synthetic resin material 14 is coated with the metal powder 15. Thus, the outer periphery of the electrode element 1 is formed with irregularities to increase the effective area. Thus, satisfactory electrode characteristics can be obtained to reduce the weight. With the weight reduction, when the electrode element 1 is held in close contact with the skin, it is held stably on the skin and difficultly detached from the skin.

Further, since the neck portion 12 made narrower than the head portion 11 and bottom portion 13 to form the engagement portion 12a, and the expansible member 2 is engaged in the engagement portion 12a, there is no need of providing any clamping members. It is thus possible to save material, simplify the manufacture and reduce the manufacturing cost.

Further, since the head portion is formed with the straight recessed portion 11a and the neck portion is formed with the straight portion continuous to and forming a straight surface with the recessed portion 11a, the material 14 may be satisfactorily coated to and even a small quantity of metal powder 15, and also the electric conductivity is improved.

Further, the electrocardiographic electrode can be reliably held in close contact with the skin of the living body by the attachment member 4 with the adhesive film 5.

Further, near the bottom portion 13 of the electrode element 1 there are provided the gell-like electrolyte material 6 and electrolyte material cover 7 covering the electrolyte material 6, thus improving the conduction of electricity with respect to the skin.

As has been described in the foregoing, according to the invention the electrode element is formed by coating the outer surface of the synthetic resin material with the metal powder, the outer surface of the electrode element is formed with irregularities to increase the effective area, so that it is possible to provide satisfactory electrode characteristics and reduce weight. With the weight reduction, when the electrocardiographic electrode is held in close contact with the skin, it is held stably and difficultly detached from the skin.

Further, since the electrode element is made of a synthetic resin material, it is possible to reduce cost by mass production. This means that it is possible to improve the sanitation by providing consummable electrocardiographic electrodes. Further, it is possible to prevent heat conduction so as to prevent the sense of coldness when the electrode is attached to the skin.

Further, since the neck portion is made narrower than the head portion and bottom portion to form the engagement portion and the expansible member is engaged in the engagement portion such that the expansible member is clamped between the head portion and bottom portion, there is no need of providing any particular clamping members, the material can be saved, the manufacture is simplified, and the cost of manufacture can be reduced.

Further, since the head portion is formed with a straight recessed portion and the neck portion is formed with a straight portion continuous to the recessed portion, even a small quantity of metal powder is satisfactorily coated on the metarial, and also the electric conductivity is improved.

Furthermore, since the attachment member having a central opening is secured to the expansible member and the adhesive film is secured to the expansible member, the electrocardiographic electrode can be stably held in close contact with the skin of the living body.

## Claims

1. Electrocardiographic electrode comprising an electrode member (1) made of synthetic resin and having a head portion (11) with a lead attached thereto, a neck portion (12) having an engagement portion (12a) narrower than the head portion and capable of being engaged in an expansible member (2) and a bottom portion (13) formed under and broader than and supporting said neck portion, the outer surface of said electrode member (1) being imparted with electric conductivity by a metal coating (15), the expansible member (2) engagedly locked in said engagement portion (12a) of the neck portion (12), an adhesive film (5) consisting of an adhesive, having a central opening and secured to said expansible member to be held in close contact with the skin of the living body, **characterized** in that said coating is made from metal powder, the head portion (11) has a straight, recessed portion (11a), and that the neck portion (12) has a straight portion (12b) continuous to and forming a straight surface with the straight recessed portion (11a) of the head portion, the bottom portion (13) being in contact with the skin of the living body through gel-like electrolyte material (6) and an electrolyte material cover (7), wherein the term "straight" qualifies the shape of the outline of a longitudinal section of the electrode member.

2. Electrocardiographic electrode comprising an electrode member (1) made of synthetic resin and having a head portion (11) with a lead attached thereto, a neck portion (12) having an engagement portion (12a) narrower than the head portion and capable of being engaged in an expansible member (2) and a bottom portion (13) formed under and broader than and supporting said neck portion, the outer surface of said electrode member (1) being imparted with electric conductivity by a metal coating (15), the expansible member (2) engagedly locked in said engagement portion (12a) of the neck portion (12), an adhesive film (5) consisting of an adhesive, having a central opening and secured to said expansible member to be held in close contact with the skin of the living body, **characterized** in that said coating is made from metal powder, the head portion (11) has a straight, recessed portion (11a), and that the neck portion (12) has a straight portion (12b) continuous to and forming a straight surface with the straight recessed portion (11a) of the head portion, the bottom portion (13) being held in contact with the skin of a living body, the lower surface area (13b) thereof being irregular to increase the contact area with the skin, wherein the term "straight" qualifies the shape of the outline of a longitudinal section of the electrode member.

## Patentansprüche

1. Elektrokardiographische Elektrode mit einem Elektrodenteil (1), das aus synthetischem Harz besteht und einen Kopfbereich (11) mit einer an diesen angeschlossenen Leitung, einen Halsbereich (12) mit einem Eingriffsbereich (12a), der schmaler als der Kopfbereich ist und in ein dehnbares Teil (2) eingreifen kann, und einen Bodenbereich (13) umfaßt, der unterhalb des Halsbereichs ausgebildet ist, breiter als dieser ist und den Halsbereich abstützt, welche äußere Oberfläche des Elektrodenteils (1) durch einen Metallüberzug (15) elektrische Leitfähigkeit erfährt, welches ausdehnbare Teil (2) in den Eingriffsbereich (12a) des Halsbereichs (12) eingreift und hier festgelegt ist, wobei ein Klebstoff-Film (5), der aus einem Klebstoff besteht, eine zentrale Öffnung aufweist und an dem ausdehnbaren Teil befestigt ist, das in engem Kontakt mit der Haut des Lebewesens zu halten ist, dadurch **gekennzeichnet,** daß der Überzug aus Metallpulver besteht, daß der Kopfbereich (11) einen geraden, ausgenommenen Bereich (11a) aufweist, und daß der Halsbereich (12) einen geraden Bereich (12b) aufweist, der durchlaufend und unter Bildung einer geraden Oberfläche in bezug auf den geraden, ausgenommenen Bereich (11a) des Kopfbereichs ausgebildet ist, wobei der Bodenbereich (13) in Berührung mit der Haut des Lebewesens über ein gelförmiges Elektrolyt-Material (6) und eine Elektrolyt-Materialabdeckung (7) steht, wobei der Ausdruck "gerade" die Form der Außenlinie eines Längsschnittes des Elektrodenteils beschreibt.

2. Elektrokardlographische Elektrode mit einem Elektrodenteil (1), das aus synthetischem Harz besteht und einen Kopfbereich (11) mit einer an diesen angeschlossenen Leitung, einen Halsbereich (12) mit einem Eingriffsbereich (12a), der schmaler als der Kopfbereich ist und in ein dehnbares Teil (2) eingreifen kann, und eInen Bodenbereich (13) umfaßt, der unterhalb des Halsbereichs ausgebildet ist, breiter als dieser ist und den Halsbereich abstützt, welche äußere Oberfläche des Elektrodenteils (1) durch einen Metallüberzug (15) elektrische Leitfähigkeit erfährt, welches ausdehnbare Teil (2) in den Eingriffsbereich (12a) des Halsbereichs (12) eingreift und hier festgelegt ist, wobei ein Klebstoff-Film (5), der aus einem Klebstoff besteht, eine zentrale Öffnung aufweist und an dem ausdehnbaren Teil befestigt ist, das in engem Kontakt mit der Haut des Lebewesens zu halten ist, dadurch **gekennzeichnet,** daß der Überzug aus Metallpulver besteht und der Kopfbereich einen geraden, ausgenommenen Bereich (11a) aufweist, und daß der Halsbereich (12) einen geraden Bereich (12b) aufweist, der durchlaufend und unter Bildung einer geraden Oberfläche in bezug auf den geraden, ausgenommenen Bereich (11a) des Kopfbereichs ausgebildet ist, wobei der Bodenbereich (13) in Berührung gehalten wird mit der Haut des Lebewesens, wobei dessen untere Oberfläche (13b) unregelmäßig geformt ist und den Berührungsbereich mit der Haut erhöht, wobei der Ausdruck "gerade" die Form der Außenlinie eines Längsschnittes des Elektrodenteils beschreibt.

## Revendications

1. Electrode pour électrocardiographie avec un membre d'électrode (1) en résine synthétique, comprenant une tête (11), à laquelle un conducteur est raccordé, une gorge (12), avec une zone d'engagement (12a), plus étroite que la tête et dans laquelle une pièce extensible (2) peut s'engager, et une base (13), qui est formée au-dessous de la gorge, plus large que celle-ci et la soutenant, un revêtement métallique (15) conférant à la surface extérieure du membre d'électrode (1) une conductibilité électrique, la pièce extensible (2) entrant en prise avec la zone d'engagement (12a) de la gorge (12), où elle est maintenue, une pellicule adhésive (5), composée de colle et présentant une ouverture centrale, est fixée à la pièce extensible, qui doit être maintenue en contact étroit avec la peau de l'homme ou de l'animal,
caractérisée par le fait
que le revêtement consiste en poudre métallique, que la tête (11) présente une zone droite, évidée (11a) et que la gorge (12) présente une zone droite (12b), conçue en continu et formant une surface droite en rapport avec la zone évidée (11a) de la tête, la base (13) étant en contact avec la peau de l'homme ou de l'animal par l'intermédiaire d'une matière électrolytique (6), genre gel, et d'un recouvrement (7) de la matière électrolytique, l'expression "droite" décrivant la forme de la ligne extérieure d'une coupe longitudinale du membre d'électrode.

2. Electrode pour électrocardiographie avec un membre d'électrode (1) en résine synthétique, comprenant une tête (11), à laquelle un conducteur est raccordé, une gorge (12), avec une zone d'engagement (12a), plus étroite que la tête et dans laquelle une pièce extensible (2) peut s'engager, et une base (13), qui est formée au-dessous de la gorge, plus large que celle-ci et la soutenant, un revêtement métallique (15) conférant à la surface extérieure du membre d'électrode (1) une conductibilité électrique, la pièce extensible (2) entrant en prise avec la zone d'engagement (12a) de la gorge (12), où elle est maintenue, une pellicule adhésive (5), composée de colle et présentant une ouverture centrale, est fixée à la pièce extensible, qui doit être maintenue en contact étroit avec la peau de l'homme ou de l'animal,
caractérisée par le fait
que le revêtement consiste en poudre métallique et que la tête présente une zone droite, évidée (11a), et que la gorge (12) présente une zone droite (12b), conçue en continu et formant une surface droite par rapport à la zone droite, évidée (11a) de la tête, la base (13) étant maintenue en contact avec la peau de l'homme ou de l'animal, sa surface inférieure (13b) étant irrégu-lière et la zone de contact avec la peau étant surélevée, l'expression "droite" décrivant la forme de la ligne extérieure d'une coupe longitudinale du membre d'électrode.
